(19) Europäisches Patentamt
European Patent Office
Office européen des brevets

(11) **EP 3 574 936 B1**

(12) **EUROPÄISCHE PATENTSCHRIFT**

(45) Veröffentlichungstag und Bekanntmachung des Hinweises auf die Patenterteilung:
**02.06.2021 Patentblatt 2021/22**

(51) Int Cl.:
*A61M 1/16* (2006.01)    *A61M 1/28* (2006.01)
*G05D 11/13* (2006.01)

(21) Anmeldenummer: **19187803.2**

(22) Anmeldetag: **15.04.2016**

(54) **VERFAHREN UND VORRICHTUNG ZUR HERSTELLUNG EINER MEDIZINISCHEN LÖSUNG**

METHOD AND DEVICE FOR THE PRODUCTION OF A MEDICAL SOLUTION

PROCÉDÉ ET DISPOSITIF DE FABRICATION D'UNE SOLUTION MÉDICALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2015 DE 102015005142**

(43) Veröffentlichungstag der Anmeldung:
**04.12.2019 Patentblatt 2019/49**

(62) Dokumentnummer(n) der früheren Anmeldung(en) nach Art. 76 EPÜ:
**16720711.7 / 3 285 825**

(73) Patentinhaber: **Fresenius Medical Care Deutschland GmbH**
**61352 Bad Homburg (DE)**

(72) Erfinder: **Kopperschmidt, Pascal**
**97456 Dittelbrunn (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A1- 2 494 998      EP-A2- 0 160 272**
**WO-A1-2014/173747      US-A1- 2015 008 183**

**Beschreibung**

Die vorliegende Erfindung betrifft ein Verfahren und eine Vorrichtung zur Herstellung einer medizinischen Lösung aus wenigstens einer ersten und wenigstens einer zweiten flüssigen Komponente, wobei die erste Komponente und die zweite Komponente mit jeweils wenigstens einem Fördermittel gefördert werden, um zumindest eine Mischlösung zu erhalten.

Aus dem Stand der Technik ist es bekannt, Dialyselösungen aus zwei Komponenten herzustellen, von denen die eine ein saures und die andere ein basisches Konzentrat ist. Beide Komponenten müssen in ihrer Konzentration überwacht werden. Dies erfolgt bei bekannten Geräten dadurch, dass für jede der Komponenten ein Leitfähigkeitssensor in Verbindung mit einer Temperaturmessung zur Berücksichtigung der Temperaturabhängigkeit der Leitfähigkeit vorgesehen ist.

Die Förderung der Komponenten, d.h. der Konzentrate erfolgt mittels Konzentratpumpen. Weichen die ermittelten Leitfähigkeitswerte von Sollwerten ab, erfolgt ein Gerätealarm.

Wie ausgeführt, werden die Konzentrationen der Komponenten, im Folgenden auch als "partielle Komponenten" bezeichnet, üblicherweise durch einzelne Leitfähigkeitssensoren erfasst, so dass eine Abweichung vom jeweiligen Sollwert unabhängig von der Summenkonzentration, d.h. von der Konzentration der die Komponenten enthaltenden Mischlösung erfasst werden kann.

Die EP 2 494 998 A1 offenbart eine Vorrichtung zur Herstellung einer medizinischen Lösung ,das sämtliche Merkmale aus dem Oberbegriff des Anspruchs 1 aufweist.

Ein Nachteil dieser bekannten Vorgehensweise besteht darin, dass für jede Komponente eine Sensorzelle benötigt wird. Ggf. wird als weiteres Schutzsystem eine weitere Sensorzelle zur Messung der Leitfähigkeit der Mischlösung eingesetzt, so dass sich insgesamt ein vergleichsweise komplexer Aufbau ergibt.

Der vorliegenden Erfindung liegt die Aufgabe zugrunde, ein Verfahren und eine Vorrichtung zur Herstellung einer medizinischen Lösung und insbesondere einer Dialyselösung, wie sie beispielsweise bei der Hämodialyse zum Einsatz kommt, gegenüber dem Stand der Technik zu vereinfachen.

Diese Aufgabe wird durch ein Verfahren mit den Merkmalen des Anspruchs 1 und durch eine Vorrichtung mit den Merkmalen des Anspruchs 8 gelöst. Erfindungsgemäß ist vorgesehen, dass die Fördermittel derart betrieben werden, dass eine Modulation der Konzentration der ersten und der zweiten Komponente erfolgt und dass an wenigstens einer, vorzugsweise an genau einer Messstelle die Leitfähigkeit der Mischlösung oder ein mit der Leitfähigkeit korrelierter Parameter der Mischlösung gemessen wird, wobei die Modulation der Konzentrationen im einem Sollzustand derart erfolgt, dass eine bestimmte Modulation (Soll-Modulation) oder keine Modulation der gemessenen Leitfähigkeit der Mischlösung oder des mit der Leitfähigkeit korrelierten Parameters der Mischlösung auftritt.

Der vorliegenden Erfindung liegt somit der Gedanke zugrunde, die Komponenten, wie z.B. das basische Konzentrat und das saure Konzentrat so zu fördern, dass die Konzentrationen der ersten Komponente und die der zweiten Komponente in der Mischlösung über die Zeit veränderlich sind.

Diese Modulation der wenigstens zwei Komponenten kann so vorgenommen werden, dass die Summenleitfähigkeit bzw. die damit korrelierte Summenkonzentration, d.h. die Leitfähigkeit oder die Konzentration der Mischlösung keine Modulation aufweist, d.h. zeitlich konstant ist oder eine ganz bestimmte Soll-Modulation aufweist.

Wird im Rahmen der Auswertung der gemessenen Leitfähigkeit der Mischlösung oder eines damit korrelierten Parameters, wie z.B. der Summenkonzentration der Mischlösung festgestellt, dass diese moduliert ist oder hinsichtlich ihrer Modulation von der bestimmten Soll-Modulation abweicht, kann auf einen Fehlerzustand geschlossen werden. Dieser kann beispielsweise darin bestehen, dass eine oder beide Komponenten mit zu hoher oder mit zu geringer Konzentration in der Mischlösung vorliegen.

Besonders vorteilhaft ist es, wenn die Modulation der Konzentrationen der ersten und der zweiten Lösung derart vorgenommen wird, dass in der Mischlösung keine zeitlichen Schwankungen der Leitfähigkeit oder der Konzentration oder eines damit korrelierten Parameters auftreten. Wird dann abweichend von diesem Sollzustand festgestellt, dass die Leitfähigkeit oder ein damit korrelierter Parameter moduliert ist, d.h. zeitlich nicht konstant ist, kann auf einen Fehlerzustand geschlossen werden.

Dementsprechend kann eine Auswertung des gemessenen Wertes der Mischlösung dahingehend vorgenommen werden, ob eine Modulation dieses Wertes vorliegt. Ist dies der Fall, kann auf einen Fehlerzustand geschlossen werden.

Entsprechendes gilt für den Fall, dass eine ganz bestimmte Soll-Modulation der Summenkonzentration, Leitfähigkeit etc. der Mischlösung eingestellt wird, die den Sollzustand darstellt, und sodann festgestellt wird, dass die tatsächliche Modulation von der bestimmten Soll-Modulation abweicht. Auch in diesem Fall kann auf einen Fehlerzustand geschlossen werden.

In einer vorteilhaften Ausgestaltung der Erfindung kann aus der Art des Fehlerzustandes auf die Komponente geschlossen werden, deren Konzentration von dem Erwartungswert abweicht. Somit wird nicht nur allgemein auf einen Fehlerzustand, d.h. auf einem vom Soll-Zustand abweichenden Zustand geschlossen, sondern es kann festgestellt werden, welche der mehreren Komponenten in einer Konzentration vorliegt, die von dem Sollwert abweicht.

An dieser Stelle wird darauf hingewiesen, dass die vorliegende Erfindung nicht auf die Verwendung von genau zwei Lösungen zur Herstellung einer Mischlösung beschränkt ist, sondern dass auch mehr als zwei Lösungen zum Einsatz kommen können.

Bei der Mischlösung kann es sich um die fertige, verabreichbare medizinische Lösung, insbesondere Dialyselösung handeln oder auch um eine Lösung, die erst nach der Zugabe einer oder mehrerer weiterer Substanzen zur fertigen medizinischen Lösung, insbesondere Dialyselösung wird.

Das erfindungsgemäße Verfahren kann durchgeführt werden, während der Patient nicht mit der Vorrichtung zur Herstellung der Lösung verbunden ist. Von der Erfindung ist auch der Fall umfasst, dass das Verfahren im Sinne einer online-Herstellung der Lösung durchgeführt wird, während der Patient an die Vorrichtung angeschlossen ist.

Bei der ersten Komponente kann es sich um ein basisches Konzentrat und bei der zweiten Komponente um ein saures Konzentrat handeln. Das basische Konzentrat weist einen pH-Wert > 7 und einen Puffer auf, das saure Konzentrat weist einen pH-Wert von < 7 und eine physiologisch verträgliche Säure auf. Eines oder beide der Konzentrate können Elektrolyte und ggf. ein Osmotikum aufweisen.

Die vorliegende Erfindung erlaubt die Überwachung der physiologischen Eigenschaften der partiellen Komponenten mit einer einzigen Leitfähigkeitsmesszelle oder mit nur einem einzigen Sensor, der zur Messung der Konzentration oder eines mit dieser bzw. mit der Leitfähigkeit korrelierten Parameters geeignet ist. Zudem kann diese Zelle bzw. der Sensor zur Überwachung der Leitfähigkeit oder der Konzentration der Mischlösung verwendet werden.

Somit besteht eine vorteilhafte Ausgestaltung der Erfindung darin, dass keine Messung der Leitfähigkeit oder eines mit der Leitfähigkeit korrelierten Parameters der ersten oder der zweiten Komponente vorgenommen wird, sondern nur die Erfassung eines Parameters, insbesondere der Leitfähigkeit der Mischlösung.

Vorzugsweise ist keine serielle Förderung der Komponenten an der Messstelle vorbei vorgesehen, da in einer bevorzugten Ausgestaltung der Erfindung auf die fehlerhaft vorliegende Komponente aus der gemessenen Eigenschaft der Mischlösung geschlossen werden kann.

Weiterhin ist erfindungsgemäß vorgesehen, dass aus einem oder mehreren der folgenden Parameter ermittelt werden kann, welche Komponente hinsichtlich ihrer Konzentration von dem Erwartungswert für diese Komponente abweicht:

- Amplitude der Modulation der gemessenen Leitfähigkeit der Mischlösung oder des mit der Leitfähigkeit korrelierten gemessenen Parameters der Mischlösung,

- mittlere gemessene Leitfähigkeit der Mischlösung oder Mittelwert des mit der Leitfähigkeit korrelierten gemessenen Parameters der Mischlösung,

- Phasenverschiebung der Modulation der gemessenen Leitfähigkeit der Mischlösung oder des mit der Leitfähigkeit korrelierten gemessenen Parameters der Mischlösung gegenüber der Anregung des Fördermittels.

Für die letztgenannte Alternative wird somit die Anregung des Aktuators bzw. des Fördermittels für die Komponente erfasst, der/das vorzugsweise als Pumpe ausgeführt ist.

In einer bevorzugten Ausgestaltung der Erfindung erfolgt die Modulation der Konzentrationen der ersten und der zweiten Komponente sinusförmig.

Die vorliegende Erfindung betrifft des Weiteren eine Vorrichtung zur Herstellung einer medizinischen Lösung, wobei die Vorrichtung wenigstens ein erstes Fördermittel zur Förderung einer ersten Komponente und wenigstens ein zweites Fördermittel zur Förderung einer zweiten Komponente aufweist sowie wenigstens eine Hauptleitung, die mit den Fördermitteln derart in Verbindung steht, dass die Komponenten durch die Fördermittel in die Hauptleitung gefördert werden, so dass in der Hauptleitung zumindest eine Mischlösung entsteht, wobei wenigstens ein, vorzugsweise genau ein Sensor zur Messung der Leitfähigkeit oder eines mit der Leitfähigkeit korrelierten Parameters der Mischlösung in der Hauptleitung vorgesehen ist und dass die Fördermittel so ausgebildet sind, dass eine Modulation der Konzentrationen im einem Sollzustand der Vorrichtung derart erfolgt, dass eine bestimmte Soll-Modulation oder keine Modulation der gemessenen Leitfähigkeit der Mischlösung oder des mit der Leitfähigkeit korrelierten gemessenen Parameters der Mischlösung auftritt.

Vorzugsweise weist die Vorrichtung wenigstens eine Auswerteeinheit auf, die mit dem Sensor in Verbindung steht und die das von dem Sensor erfasste Signal auswertet, wobei die Auswerteeinheit derart ausgebildet ist, dass auf einen Fehlerzustand geschlossen wird, wenn eine Modulation oder eine von der Soll-Modulation abweichende Modulation der gemessenen Leitfähigkeit der Mischlösung oder des mit der Leitfähigkeit korrelierten gemessenen Parameters der Mischlösung festgestellt wird.

Die Auswerteeinheit kann derart ausgebildet sein, dass aus der Art des Fehlerzustandes ermittelbar ist, welche Komponente hinsichtlich ihrer Konzentration von dem Erwartungswert abweicht.

Wie oben ausgeführt, ist vorzugsweise genau ein Sensor zur Messung der Leitfähigkeit oder eines mit der Leitfähigkeit korrelierten Parameters der Mischlösung vorgesehen.

Auf Sensoren zur Messung der individuellen Leitfähigkeit oder eines mit der Leitfähigkeit korrelierten individuellen Parameters der ersten oder der zweiten Komponente wird vorzugsweise verzichtet.

Die Auswerteeinheit ist erfindungsgemäß weiterhin derart ausgebildet, dass aus der Amplitude der Modulation der gemessenen Leitfähigkeit oder des mit der Leitfähigkeit korrelierten Parameters und/oder aus der mittleren gemessenen Leitfähigkeit oder aus dem Mittelwert des mit der Leitfähigkeit korrelierten Parameters und/oder aus der Phasenverschiebung der Modulation der gemessenen Leitfähigkeit oder des mit der Leitfähigkeit korrelierten Parameters gegenüber der Anregung des Fördermittels bestimmt wird, welche Komponente hinsichtlich ihrer Konzentration von dem Erwartungswert für diese Komponente abweicht.

Für den Vergleich mit der Modulation des Fördermittels ist ein entsprechender Sensor oder ein sonstiges Erfassungsmittel vorgesehen, der diese Modulation, d.h. die Anregung der Pumpe oder dergleichen erfasst.

In einer bevorzugten Ausführungsform erfolgt die Modulation der Konzentration der ersten und der zweiten Komponente sinusförmig.

Die vorliegende Erfindung betrifft des Weiteren eine Blutbehandlungsvorrichtung, insbesondere eine Dialysemaschine, vorzugsweise zur Durchführung einer Hämodialysebehandlung, wobei die Blutbehandlungsvorrichtung wenigstens eine erfindungsgemäße Vorrichtung zur Herstellung der medizinischen Lösung aufweist.

Weitere Einzelheiten und Vorteile der Erfindung werden anhand eines in der Zeichnung dargestellten Ausführungsbeispiels näher erläutert. Es zeigen:

Figur 1: die Summenleitfähigkeit der Komponenten A und B sowie den Beitrag der Einzelleitfähigkeiten der Komponenten A und B zur Summenleitfähigkeit,

Figur 2: die Summenleitfähigkeit der Komponenten A und B sowie den Beitrag der Einzelleitfähigkeiten der Komponenten A und B zur Summenleitfähigkeit für vier verschiedene Zustände A bis C und

Figur 3: eine schematische Ansicht eines Multikomponentensystems umfassend die Komponenten 1 bis N mit einer Leitfähigkeitsmesszelle zur Messung der Summenleitfähigkeit der Mischlösung.

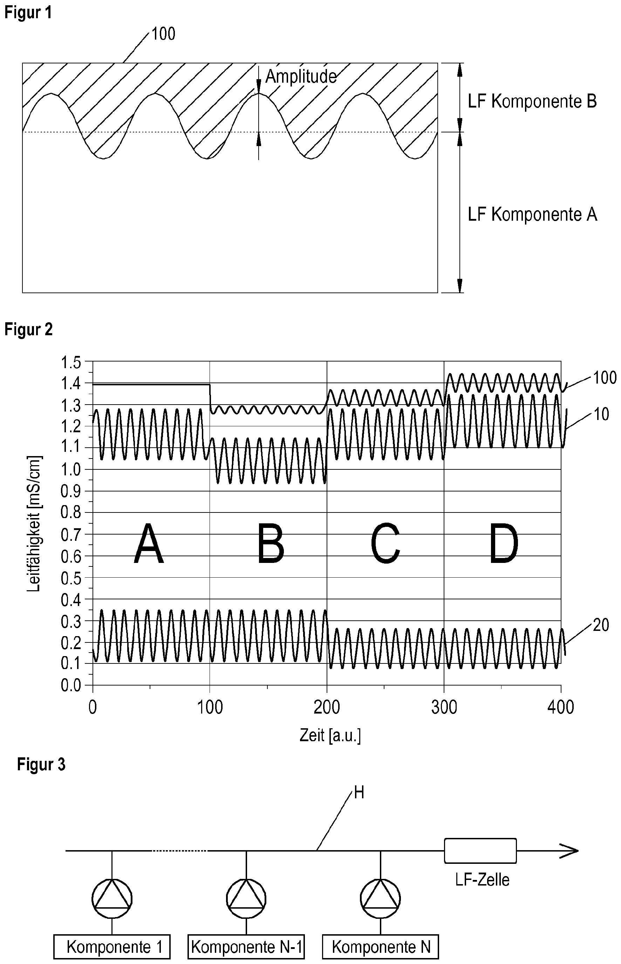

Aus Figur 1 ist der schematische zeitliche Verlauf der Summenleitfähigkeit 100, d.h. der Leitfähigkeit der aus dem den Komponenten A und B zusammengesetzten Mischlösung über die Zeit ersichtlich. Des Weiteren sind die zeitlichen Verläufe der Leitfähigkeiten der Komponenten A und B (LF Komponente A, LF Komponente B) so dargestellt, dass der Wert für die Leitfähigkeit der Komponente B (gestrichelt dargestellt) zu dem Wert der Leitfähigkeit der Komponente A hinzuaddiert wird, so dass sich eine zeitlich konstante Summenleitfähigkeit 100 ergibt.

Das Ausführungsbeispiel bezieht sich auf die Messung der Leitfähigkeit und ist auch für die Messung jedes anderen Parameters denkbar. Die Erfindung umfasst somit auch die Messung jedes anderen Parameters der Mischlösung, der mit der Leitfähigkeit bzw. mit der Konzentration korreliert, so dass ein Rückschluss auf die Konzentration der Komponenten A und/oder B bzw. auf deren Inhaltsstoffe gezogen werden kann.

Aus Figur 1 ist ersichtlich, dass die Pumpenförderung der Komponenten A und B derart moduliert wird, dass die Summenleitfähigkeit 100 der Mischung keine Modulation aufweist, d.h. zeitlich konstant ist. Die Summenkonzentration, die mit der Summenleitfähigkeit korreliert ist, ist somit zeitlich konstant, wohingegen die Konzentrationen der Komponenten A und B in der Mischlösung aus beiden Komponenten zeitlich variieren.

Die Förderung der partiellen Komponenten A und B erfolgt in dem in Figur 1 dargestellten Ausführungsbeispiel um $\pi$ phasenverschoben und phasenkonstant moduliert, so dass die Summenkonzentration konstant ist bzw. die Summenleitfähigkeit keine Schwankungen über die Zeit aufweist.

Die Amplitude der Konzentrations- bzw. Leitfähigkeitsschwankungen, die in Figur 1 eingezeichnet ist, ist für die beiden Komponenten A und B identisch.

Geht man davon aus, dass die partiellen Komponenten, d.h. die Komponenten A und B, die die Mischlösung bilden bzw. in dieser ggf. in einem Lösungsmittel, wie insbesondere Wasser vorliegen, in der Mischlösung die Konzentrationen $C_A$ und $C_B$ aufweisen und die Einzelleitfähigkeiten bzw. Leitfähigkeitsbeiträge der Komponenten A und B in der Mischlösung $LF_A(t)$ und $LF_B(t)$ sind, ergibt sich bei den relativen Amplituden $f_A$ und $f_B$ und einer co-Modulation für den zeitlichen Verlauf der Leitfähigkeiten:

$$LF_A(t) = C_A \left(1 + f_A \sin(\omega t)\right) \qquad (1)$$

$$LF_B(t) = C_B \left(1 + f_B \sin(\omega t)\right) \qquad (2)$$

Die relative Amplitude ist der Quotient aus der absoluten Amplitude der Konzentrationsschwankung und dem Mittelwert der Konzentration der Komponente A bzw. B in der Mischlösung.

Aus der vorzugsweise angestrebten Freiheit von Modulationen der Summenkonzentration bzw. der Summenleitfähigkeit über die Zeit ergibt sich:

$$C_A \, f_A = - \, C_B \, f_B \qquad (3)$$

und aufgelöst nach $f_A$:

$$f_A = - \, f_B \, (C_B / C_A) \qquad (4)$$

Legt man die relative Amplitude der Modulation der Komponente B z.B. auf den Wert 0,5 ergibt sich weiter:

$$f_A = - \, 0{,}5 \, (C_B / C_A) = - \, 0{,}5 \, f_{AB} \qquad (5)$$

mit $f_{AB} = C_B / C_A$.

Damit ergibt sich die relative Amplitude $f_A$ der Komponente A unmittelbar aus dem angestrebten Verhältnis der Konzentrationen der Komponenten B und A in der Mischlösung und der relativen Amplitude $f_B$ der Komponente B.

Die gemessene Summenleitfähigkeit, d.h. die Leitfähigkeit der aus dem Einzelkomponenten A und B zusammengesetzten Mischlösung wird im Sollzustand den gewünschten physiologischen Erwartungswert aufweisen bzw. mit der erwarteten Summenkonzentration, d.h. der Summe der Konzentration der Einzelkomponenten A und B korrelieren. Für das oben genannte Beispiel mit $f_B = 0{,}5$ ergibt sich dann aus den Gleichungen (1), (2) und (5):

$$LF_A(t) + LF_B(t) = C_A \, (1 - 0{,}5 \, (C_B / C_A) \sin(\omega t)) + C_B \, (1 + 0{,}5 \sin(\omega t)) = C_A + C_B \qquad (6)$$

Die Erwartungswerte $C_A$ und $C_B$ in der Mischlösung sind zeitlich konstant, so dass sich auch hinsichtlich der Summenleitfähigkeit $LF_A(t) + LF_B(t)$ eine zeitliche Konstanz ergibt.

Weicht der Beitrag einer Komponente A oder B von dem jeweiligen Erwartungswert ab, kommt es zu einer Modulation der Summenleitfähigkeit. Je nachdem, welche Komponente hinsichtlich der Konzentration von dem Erwartungswert abweicht, wird eine bestimmte Modulation der Summenleitfähigkeit erzeugt, d.h. die Messung der Summenleitfähigkeit erzeugt einen charakteristischen Fingerabdruck. Somit kann anhand der Messung der Summenleitfähigkeit festgestellt werden, welche Komponente vom Erwartungswert abweicht.

Geht man davon aus, dass die Komponente B hinsichtlich ihrer Konzentration in der Mischlösung nicht dem Erwartungswert $C_B$ entspricht, sondern nur den Wert $C_B'$ aufweist, der einen bestimmten Bruchteil $1/a$ des Erwartungswertes $C_B$ darstellt, ergibt sich:

$$C_B' = 1/\alpha \, C_B \qquad (7)$$

Die Summenleitfähigkeit weist somit in diesem Fall nicht den sich aus Gleichung (6) ergebenden Wert auf, sondern ergibt sich unter Berücksichtigung von Gleichung (5) und (7) zu:

$$LF_A(t) + LF_B(t) = C_A \, (1 - 0{,}5 \, (C_B / C_A) \sin(\omega t)) + C_B' \, (1 + 0{,}5 \sin(\omega t)) \qquad (8)$$

$$= C_A + 1/\alpha \, C_B + (1/\alpha - 1) \, C_B / 2 \, \sin(\omega t) \qquad (9)$$

Weicht somit der Komponentenbeitrag der Komponente B um den Faktor $1/a$ vom Erwartungswert ab, so moduliert die Leitfähigkeit der Summenkonzentration, d.h. der Mischlösung aus den beiden Komponenten A und B in diesem Ausführungsbeispiel mit der Amplitude $(1/\alpha - 1) \, C_B / 2$.

Die mittlere Summenleitfähigkeit entspricht bzw. korreliert in diesem Fall mit $C_A + 1/a \, C_B$.

Geht man davon aus, dass die Komponente A hinsichtlich ihrer Konzentration nicht dem Erwartungswert $C_A$ entspricht, sondern nur den Wert $C_A'$ aufweist, der einen bestimmten Bruchteil $1/\beta$ des Erwartungswertes $C_A$ darstellt, ergibt sich:

$$C_A{}' = 1/\beta\ C_A \qquad (10)$$

Die Summenleitfähigkeit weist somit in diesem Fall nicht den sich aus Gleichung (6) ergebenden Wert auf, sondern ergibt sich unter Berücksichtigung von Gleichung (5) und (10) zu:

$$LF_A(t) + LF_B(t) = 1/\beta\ C_A\ (1 - 0{,}5\ (C_B/C_A)\ \sin(\omega t)) + C_B\ (1 + 0{,}5\ \sin(\omega t)) \qquad (11)$$

$$= 1/\beta\ C_A + C_B + (1 - 1/\beta)\ C_B/2\ \sin(\omega t) \qquad (12)$$

Weicht somit der Komponentenbeitrag der Komponente A um den Faktor $1/\beta$ vom Erwartungswert ab, so moduliert die Leitfähigkeit der Summenkonzentration, d.h. der Mischlösung aus den beiden Komponenten A und B in diesem Ausführungsbeispiel mit der Amplitude $(1 - 1/\beta)\ C_B/2$.

Die mittlere Summenleitfähigkeit entspricht bzw. korreliert in diesem Fall mit $1/\beta\ C_A + C_B$.

Die mittlere Leitfähigkeit, deren Amplitude und die Phase gegenüber der Aktuatoranregung der Komponentenförderung identifizieren die Komponente, deren Konzentration von dem Erwartungswert abweicht.

Leisten die Komponenten zur Summenkonzentration einen unterschiedlichen Beitrag, kann auch eine sich kompensierende Abweichung unter den Komponenten identifiziert werden.

Figur 2 zeigt in dem Zustand A den Fall, dass die Erwartungswerte der Beiträge der Komponenten A und B erreicht ist. In diesem Fall zeigt die Summenleitfähigkeit 100 bzw. die Summenkonzentration der Mischlösung keine Modulation, d.h. ist zeitlich konstant. Die Bezugszeichen 10 und 20 kennzeichnen den zeitlichen Verlauf der Beiträge der Leitfähigkeit oder der Konzentration der partiellen Komponenten A (Bezugszeichen 10) und B (Bezugszeichen 20) zur Leitfähigkeit 100 oder Konzentration der Mischlösung.

In dem Zustand B ist der Erwartungswert 20 der Komponente B erreicht, jedoch ist der tatsächliche Wert der Konzentration 10 der Komponente A unter dem Erwartungswert. Die Summenleitfähigkeit 100 bzw. die Summenkonzentration liegt unter dem Erwartungswert und ist phasenverschoben moduliert mit der abweichenden Komponente A.

In dem Zustand C ist der Erwartungswert 10 der Komponente A erreicht, jedoch ist der tatsächliche Wert der Konzentration 20 der Komponente B unter dem Erwartungswert. Die Summenleitfähigkeit 100 bzw. die Summenkonzentration liegt unter dem Erwartungswert und ist phasenverschoben moduliert mit der abweichenden Komponente B.

In dem Zustand D sind beide Erwartungswerte der Komponenten A und B nicht erreicht. Die Abweichung ist jedoch kompensatorisch, d.h. der Summenbeitrag entspricht dem Erwartungswert, d.h. der Mittelwert der Summenleitfähigkeit 100 bzw. der Summenkonzentration entspricht dem Erwartungswert.

Allerdings ist auch in diesem Fall die Summenleitfähigkeit 100 bzw. die Summenkonzentration moduliert, d.h. zeitlich nicht konstant.

Eine Abweichung der Konzentrationsbeiträge der Komponenten A und B lässt sich somit aus der Analyse der modulierten Summenkonzentration bzw. Summenleitfähigkeit vornehmen.

Aus dieser Analyse ergibt sich die Phasenverschiebung der Modulation der Summenkonzentration bzw. der Summenleitfähigkeit gegenüber den Phasen der Konzentratförderpumpen.

Der mittlere Erwartungswert der Summenkonzentration bzw. der Summenleitfähigkeit weist auf eine Über- oder Unterförderung der abweichenden Komponente hin, d.h. der gemessene Mittelwert der Summenleitfähigkeit oder der Summenkonzentration weist auf eine Über- oder Unterförderung der abweichenden Komponente hin.

So lässt sich beispielsweise aus dem Zustand B in Figur 2 nicht nur entnehmen, dass die Komponente A mit einer von dem Erwartungswert abweichenden Konzentration vorliegt, sondern auch, dass hinsichtlich dieser Komponente eine Unterförderung vorliegt.

Figur 3 zeigt ein Multikomponentensystem mit modulierter Förderung der Konzentrationen der Komponenten 1 bis N. Sämtliche Komponenten werden in eine Hauptleitung H gefördert, in der sich die einzige Leitfähigkeitsmesszelle (LF-Zelle) befindet. Die jeweiligen Modulationen sind hinsichtlich Frequenz und Phase charakteristisch für jede Komponente. Dieses Charakteristikum erlaubt eine Identifikation des vom Erwartungswert abweichenden Beitrages einer Komponente.

Über die Ermittlung der Phasenverschiebung der Amplitude der Summenleitfähigkeit oder der Summenkonzentration zur Phase der Konzentratförderpumpe kann selbst eine kompensatorische Abweichung der Komponenten ermittelt werden, die nicht zu einer Änderung der mittleren Summenleitfähigkeit oder Summenkonzentration führt.

Erfindungsgemäß ist für die Überwachung der Beiträge der einzelnen, d.h. der partiellen Komponenten lediglich ein einzelner bzw. ein einziger Leitfähigkeits- oder Konzentrationssensor oder dergleichen erforderlich. Die Komponenten werden über moduliert fördernde Konzentratpumpen der Hauptleitung zugegeben, in der sich der Sensor befindet.

Anstatt eines Leitfähigkeitssensors oder eines Konzentrationssensors kann auch jeder andere Sensor verwendet werden, der einen Rückschluss auf die Konzentrationen oder Leifähigkeiten der Komponenten bzw. auf die Summenkonzentration oder auf die Summenleitfähigkeit erlaubt.

## Patentansprüche

1. Verfahren zur Herstellung einer medizinischen Lösung aus wenigstens einer ersten und wenigstens einer zweiten Komponente, wobei die erste Komponente und die zweite Komponente als Flüssigkeiten vorliegen und mit jeweils einem Fördermittel gefördert werden, um eine Mischlösung zu erhalten, wobei die Fördermittel derart betrieben werden, dass eine Modulation der Konzentrationen der ersten und der zweiten Komponente erfolgt und dass an einer Messstelle die Leitfähigkeit oder ein mit der Leitfähigkeit korrelierter Parameter der Mischlösung gemessen wird, und wobei die Modulation der Konzentrationen der Komponenten in einem Sollzustand derart erfolgt, dass eine bestimmte Soll-Modulation oder keine Modulation der gemessenen Leitfähigkeit oder des mit der Leitfähigkeit korrelierten Parameters der Mischlösung auftritt,
**dadurch gekennzeichnet,**
**dass** aus der Amplitude der Modulation der gemessenen Leitfähigkeit der Mischlösung oder des mit der Leitfähigkeit korrelierten gemessenen Parameters der Mischlösung und/oder aus der mittleren gemessenen Leitfähigkeit der Mischlösung oder aus dem Mittelwert des mit der Leitfähigkeit korrelierten gemessenen Parameters der Mischlösung und/oder aus der Phasenverschiebung der Modulation der gemessenen Leitfähigkeit der Mischlösung oder des mit der Leitfähigkeit korrelierten gemessenen Parameters der Mischlösung gegenüber der Anregung des Fördermittels bestimmt wird, welche Komponente hinsichtlich ihrer Konzentration von dem Erwartungswert für diese Komponente abweicht.

2. Verfahren nach Anspruch 1, **dadurch gekennzeichnet, dass** eine Auswertung der gemessenen Leitfähigkeit oder des gemessenen Wertes des mit der Leitfähigkeit korrelierten Parameters der Mischlösung vorgenommen wird und bei Auftreten einer Modulation oder bei einer Abweichung von der Soll-Modulation auf einen Fehlerzustand geschlossen wird.

3. Verfahren nach Anspruch 2, **dadurch gekennzeichnet, dass** aus der Art des Fehlerzustandes auf die Komponente geschlossen wird, deren Konzentration von dem Erwartungswert für diese Komponente abweicht.

4. Verfahren nach einem der vorhergehenden Ansprüche, **dadurch gekennzeichnet, dass** es sich bei der medizinischen Lösung um eine Dialyselösung handelt, und/oder dass es sich bei der ersten Komponente um ein basisches Konzentrat und bei der zweiten Komponente um ein saures Konzentrat handelt.

5. Verfahren nach einem der vorhergehenden Ansprüche, **dadurch gekennzeichnet, dass** keine Messung der Leitfähigkeit oder eines mit der Leitfähigkeit korrelierten Parameters der ersten oder der zweiten Komponente vorgenommen wird.

6. Verfahren nach einem der vorhergehenden Ansprüche, **dadurch gekennzeichnet, dass** keine serielle Förderung der Komponenten an der Messstelle vorbei erfolgt.

7. Verfahren nach einem der vorhergehenden Ansprüche, **dadurch gekennzeichnet, dass** die Modulation der Konzentrationen der ersten und der zweiten Komponente sinusförmig erfolgt.

8. Vorrichtung zur Herstellung einer medizinischen Lösung, wobei die Vorrichtung ein erstes Fördermittel zur Förderung einer ersten Komponente und ein zweites Fördermittel zur Förderung einer zweiten Komponente aufweist sowie eine Hauptleitung, die mit den Fördermitteln derart in Verbindung steht, dass die Komponenten durch die Fördermittel in die Hauptleitung gefördert werden, so dass in der Hauptleitung eine Mischlösung entsteht, wobei ein Sensor zur Messung der Leitfähigkeit oder eines mit der Leitfähigkeit korrelierten Parameters der Mischlösung in der Hauptleitung vorgesehen ist, und wobei die Fördermittel so ausgebildet sind, dass eine Modulation der Konzentrationen der Komponenten in einem Sollzustand derart erfolgt, dass keine Modulation oder eine bestimmte Soll-Modulation der gemessenen Leitfähigkeit oder des mit der Leitfähigkeit korrelierten Parameters der Mischlösung auftritt,
**dadurch gekennzeichnet,**
**dass** die Vorrichtung eine Auswerteeinheit aufweist, die derart ausgebildet ist, dass aus der Amplitude der Modulation der gemessenen Leitfähigkeit der Mischlösung oder des mit der Leitfähigkeit korrelierten gemessenen Parameters der Mischlösung und/oder aus der mittleren gemessenen Leitfähigkeit der Mischlösung oder aus dem Mittelwert des mit der Leitfähigkeit korrelierten gemessenen Parameters der Mischlösung und/oder aus der Phasenverschiebung der Modulation der gemessenen Leitfähigkeit der Mischlösung oder des mit der Leitfähigkeit korrelierten gemessenen Parameters der Mischlösung gegenüber der Anregung des Fördermittels bestimmt wird, welche Komponente hinsichtlich ihrer Konzentration von dem Erwartungswert für diese Komponente abweicht.

9. Vorrichtung nach Anspruch 8, **dadurch gekennzeichnet, dass** die Vorrichtung eine Auswerteeinheit aufweist, der das von dem Sensor erfasst Signal zugeführt wird, wobei die Auswerteeinheit derart ausgebildet ist, dass auf einen Fehlerzustand geschlossen wird, wenn eine Modulation oder eine von der Soll-Modulation abweichende Modulation der gemessenen Leitfähigkeit oder des mit der Leitfähigkeit korrelierten Parameters der Mischlösung festgestellt wird.

10. Vorrichtung nach Anspruch 9, **dadurch gekennzeichnet, dass** die Auswerteeinheit derart ausgebildet ist, dass aus der Art des Fehlerzustandes ermittelbar ist, welche Komponente hinsichtlich ihrer Konzentration von dem Erwartungswert für diese Komponente abweicht.

11. Vorrichtung nach einem der Ansprüche 8 bis 10, **dadurch gekennzeichnet, dass** genau ein Sensor zur Messung der Leitfähigkeit oder eines mit der Leitfähigkeit korrelierten Parameters der Mischlösung vorgesehen ist.

12. Vorrichtung nach einem der Ansprüche 8 bis 11, **dadurch gekennzeichnet, dass** kein Sensor zur Messung der Leitfähigkeit oder eines mit der Leitfähigkeit korrelierten Parameters der ersten oder der zweiten Komponente vorgesehen ist und/oder dass die Fördermittel derart ausgebildet sind, dass die Modulation der Konzentrationen der ersten und der zweiten Komponente sinusförmig erfolgt.

13. Blutbehandlungsgerät, insbesondere Dialysegerät, mit einer Vorrichtung nach einem der Ansprüche 8 bis 12.

**Claims**

1. Method for producing a medical solution from at least one first and at least one second component, wherein the first component and the second component are present as liquids and are each conveyed by a conveying means in order to obtain a mixed solution, wherein the conveying means are operated in such a way that modulation of the concentrations of the first and the second component takes place and that the conductivity, or a parameter correlated with the conductivity, of the mixed solution is measured at a measuring point, and wherein the modulation of the concentrations of the components in a target state takes place such that a specific target modulation or no modulation of the measured conductivity, or of the parameter correlated with the conductivity, of the mixed solution occurs, **characterised in that** from the amplitude of the modulation of the measured conductivity of the mixed solution or of the measured parameter, correlated with the conductivity, of the mixed solution, and/or from the mean measured conductivity of the mixed solution or from the mean value of the measured parameter, correlated with the conductivity, of the mixed solution and/or from the phase shift of the modulation of the measured conductivity of the mixed solution or of the measured parameter, correlated with the conductivity, of the mixed solution compared with the excitation of the conveying means, it is determined which component deviates in respect of its concentration from the expected value for this component.

2. Method according to claim 1, **characterised in that** an evaluation of the measured conductivity or the measured value of the parameter, correlated with the conductivity, of the mixed solution is carried out and in the event of modulation occurring or in the event of a deviation from the target modulation, a fault state is deduced.

3. Method according to claim 2, **characterised in that** the component with a concentration deviating from the expected value for this component is deduced from the nature of the fault state.

4. Method according to any one of the preceding claims, **characterised in that** the medical solution is a dialysis solution, and/or that the first component is an alkaline concentrate and the second component is an acidic concentrate.

5. Method according to any one of the preceding claims, **characterised in that** no measurement of the conductivity, or of a parameter correlated with the conductivity, of the first or second component is carried out.

6. Method according to any one of the preceding claims, **characterised in that** no serial conveying of the components past the measuring point takes place.

**7.** Method according to any one of the preceding claims, **characterized in that** the modulation of the concentrations of the first and the second component takes place sinusoidally.

**8.** Device for producing a medical solution, wherein the device comprises a first conveying means for conveying a first component and a second conveying means for conveying a second component as well as a main line, which is connected to the conveying means such that the components are conveyed by the conveying means into the main line, so that a mixed solution is created in the main line, wherein a sensor is provided in the main line for measuring the conductivity, or a parameter correlated with the conductivity, of the mixed solution, and wherein the conveying means are formed such that a modulation of the concentrations of the components in a target state takes place such that no modulation or a specific target modulation of the measured conductivity, or of the parameter correlated with the conductivity, of the mixed solution occurs,
**characterised in that**
the device comprises an evaluation unit, which is formed such that from the amplitude of the modulation of the measured conductivity of the mixed solution or of the measured parameter, correlated with the conductivity, of the mixed solution, and/or from the mean measured conductivity of the mixed solution or from the mean value of the measured parameter, correlated with the conductivity, of the mixed solution and/or from the phase shift of the modulation of the measured conductivity of the mixed solution or of the measured parameter, correlated with the conductivity, of the mixed solution compared with the excitation of the conveying means, it is determined which component deviates in respect of its concentration from the expected value for this component.

**9.** Device according to claim 8, **characterised in that** the device comprises an evaluation unit to which the signal detected by the sensor is supplied, wherein the evaluation unit is formed such that a fault state is deduced if a modulation, or a modulation deviating from the target modulation, of the measured conductivity, or of the parameter correlated with the conductivity, of the mixed solution is established.

**10.** Device according to claim 9, **characterised in that** the evaluation unit is formed such that from the nature of the fault state it can be determined which component deviates in respect of its concentration from the expected value for this component.

**11.** Device according to one of claims 8 to 10, **characterised in that** precisely one sensor is provided for measuring the conductivity, or a parameter correlated with the conductivity, of the mixed solution.

**12.** Device according to any one of claims 8 to 11, **characterised in that** no sensor is provided for measuring the conductivity, or a parameter correlated with the conductivity, of the first or the second component and/or that the conveying means are formed such that the modulation of the concentrations of the first and the second component takes place sinusoidally.

**13.** Blood treatment apparatus, in particular dialysis apparatus, comprising a device according to any one of claims 8 to 12.


**Revendications**

**1.** Procédé pour fabriquer une solution médicale à partir d'au moins un premier et d'au moins un deuxième composant, dans lequel le premier composant et le deuxième composant sont présents sous la forme de liquides et sont transportés avec respectivement un moyen de transport pour obtenir une solution mélangée, dans lequel les moyens de transport fonctionnent de telle manière qu'une modulation des concentrations du premier et du deuxième composant est effectuée et que la conductivité ou un paramètre, en corrélation avec la conductivité, de la solution mélangée est mesurée ou mesurée sur un emplacement de mesure, et dans lequel la modulation des concentrations des composants est effectuée dans un état théorique de telle manière qu'une modulation théorique définie ou qu'aucune modulation de la conductivité mesurée ou du paramètre, en corrélation avec la conductivité, de la solution mélangée apparaît ou n'apparaît,
**caractérisé en ce**
**qu'**est défini, à partir de l'amplitude de la modulation de la conductivité mesurée de la solution mélangée ou du paramètre mesuré, en corrélation avec la conductivité, de la solution mélangée et/ou à partir de la conductivité mesurée moyenne de la solution mélangée ou à partir de la valeur moyenne du paramètre mesuré, en corrélation avec la conductivité, de la solution mélangée et/ou à partir du décalage de phases de la modulation de la conductivité mesurée de la solution mélangée ou du paramètre mesuré, en corrélation avec la conductivité, de la solution mélangée, par rapport à l'excitation du moyen de transport, le composant, qui diverge, quant à sa concentration, de la valeur attendue pour ledit composant.

2. Procédé selon la revendication 1, **caractérisé en ce qu'**une évaluation de la conductivité mesurée ou de la valeur mesurée du paramètre, en corrélation avec la conductivité, de la solution mélangée est réalisée et qu'un état erroné est conclu en cas d'apparition d'une modulation ou dans le cas d'une divergence par rapport à la modulation théorique.

3. Procédé selon la revendication 2, **caractérisé en ce qu'**est conclu, à partir du type de l'état erroné, le composant, dont la concentration diverge de la valeur attendue pour ledit composant.

4. Procédé selon l'une quelconque des revendications précédentes, **caractérisé en ce que** la solution médicale est une solution de dialyse, et que le premier composant est un concentré basique et le deuxième composant est un concentré acide.

5. Procédé selon l'une quelconque des revendications précédentes, **caractérisé en ce qu'**aucune mesure de la conductivité ou d'un paramètre, en corrélation avec la conductivité, du premier ou du deuxième composant n'est réalisée.

6. Procédé selon l'une quelconque des revendications précédentes, **caractérisé en ce qu'**aucun transport en série des composants le long de l'emplacement de mesure n'est effectué.

7. Procédé selon l'une quelconque des revendications précédentes, **caractérisé en ce que** la modulation des concentrations du premier et du deuxième composant est effectuée de manière sinusoïdale.

8. Dispositif pour fabriquer une solution médicale, dans lequel le dispositif présente un premier moyen de transport pour transporter un premier composant et un deuxième moyen de transport pour transporter un deuxième composant ainsi qu'un conduit principal, qui est relié aux moyens de transport de telle manière que les composants sont transportés dans le conduit principal par les moyens de transport de sorte que se forme dans le conduit principal une solution mélangée, dans lequel un capteur est prévu pour mesurer la conductivité ou un paramètre, en corrélation avec la conductivité, de la solution mélangée dans le conduit principal, et dans lequel les moyens de transport sont réalisés de telle sorte qu'une modulation des concentrations des composants est effectuée dans un état théorique de telle manière qu'aucune modulation ou une modulation théorique définie de la conductivité mesurée ou du paramètre, en corrélation avec la conductivité, de la solution mélangée, ne se produit ou se produit,
**caractérisé en ce que**
le dispositif présente une unité d'évaluation qui est réalisée de telle manière qu'est défini, à partir de l'amplitude de la modulation de la conductivité mesurée de la solution mélangée ou du paramètre mesuré, en corrélation avec la conductivité, de la solution mélangée et/ou à partir de la conductivité mesurée moyenne de la solution mélangée ou à partir de la valeur moyenne du paramètre mesuré, en corrélation avec la conductivité, de la solution mélangée et/ou à partir du décalage de phases de la modulation de la conductivité mesurée de la solution mélangée ou du paramètre mesuré, en corrélation avec la conductivité, de la solution mélangée, par rapport à l'excitation du moyen de transport, le composant qui diverge, quant à sa concentration, de la valeur attendue pour ledit composant.

9. Dispositif selon la revendication 8, **caractérisé en ce que** le dispositif présente une unité d'évaluation, à laquelle le signal détecté par le capteur est amené, dans lequel l'unité d'évaluation est réalisée de telle manière qu'un état erroné est conclu quand une modulation ou une modulation, divergeant de la modulation théorique, de la conductivité mesurée ou du paramètre, en corrélation avec la conductivité, de la solution mélangée est constatée.

10. Dispositif selon la revendication 9, **caractérisé en ce que** l'unité d'évaluation est réalisée de telle manière que peut être déterminé, à partir du type de l'état erroné, le composant, qui diverge, quant à sa concentration, de la valeur attendue pour ledit composant.

11. Dispositif selon l'une quelconque des revendications 8 à 10, **caractérisé en ce qu'**est prévu précisément un capteur pour mesurer la conductivité ou un paramètre, en corrélation avec la conductivité, de la solution mélangée.

12. Dispositif selon l'une quelconque des revendications 8 à 11, **caractérisé en ce qu'**aucun capteur n'est prévu pour mesurer la conductivité ou un paramètre, en corrélation avec la conductivité, du premier ou du deuxième composant, et/ou que les moyens de transport sont réalisés de telle manière que la modulation des concentrations du premier et du deuxième composant est effectuée de manière sinusoïdale.

13. Appareil de traitement du sang, en particulier appareil de dialyse, avec un dispositif selon l'une quelconque des revendications 8 à 12.

**Figur 1**

Amplitude

LF Komponente B

LF Komponente A

**Figur 2**

**Figur 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2494998 A1